(19) Europäisches Patentamt
European Patent Office
Office européen des brevets

(11) Veröffentlichungsnummer: **0 001 812**
**A1**

(12) # EUROPÄISCHE PATENTANMELDUNG

(21) Anmeldenummer: 78101243.0

(22) Anmeldetag: 27.10.78

(51) Int. Cl.[2]: C 07 G 7/00
G 01 N 33/16

(30) Priorität: 27.10.77 US 846089
28.08.78 US 937662

(43) Veröffentlichungstag der Anmeldung: 16.05.79 Patentblatt 79/10

(84) Benannte Vertragsstaaten: CH DE FR GB NL

(71) Anmelder: F.Hoffmann-La Roche & Co. Aktiengesellschaft
Abt. VIII-Pt
CH-4002 Basel(CH)

(72) Erfinder: Newman, Edward Samuel
River Road 9, Apt. L
Nutley New Jersey 07110(US)

(74) Vertreter: Lederer, Franz, Dr. et al,
Patentanwälte Lederer, Meyer Lucile-Grahn-Strasse 22
D-8000 München 80(DE)

(54) **Verfahren zur Herstellung gereinigten Alpha-1-Fetoproteins und dessen Verwendung.**

(57) Die Erfindung betrifft ein Verfahren zur Herstellung gereinigten $\alpha_1$-Fetoproteins bei welchem folgende Verfahrensstufen angewandt werden:

a) Puffern einer Probe eines Materials aus einer biologischen Quelle, das dafür bekannt ist, $\alpha_1$-Fetoprotein zu enthalten, auf einen pH von etwa 7,

b) Affinitätschromatographie der gepufferten Probe durch Hindurchführen durch eine Säule mit einem für $\alpha_1$-Fetoprotein spezifischen Antikörper, der auf einem festen Träger unbeweglich verankert ist, und Eluieren des gebundenen $\alpha_1$-Fetoproteins durch Behandeln der Säule mit einem geeigneten Desorptionsmittel,

c) Entfernen praktisch des gesamten, im Eluat der Stufe b) vorhandenen Albumins mit Hilfe der Adorptionschromatographie durch Hindurchleiten durch eine Säule, die einen immobilisierten Reaktivfarbstoff-Adsorber enthält,

d) Dialysieren der das nicht-adsorbierte $\alpha_1$-Fetoprotein enthaltenden Lösung der Stufe c),

e) Lyophilisieren der dialysierten Lösung der Stufe d),

f) Lösen des in Stufe e) gebildeten Pulvers in einem geeigneten Puffer und Polyacrylamid-Gelelektrophorese der erhaltenen Lösung.

Die Erfindung betrifft auch das radioaktiv jodierte, so hergestellte $\alpha_1$-Fetoprotein, ferner die Verwendung des $\alpha_1$-Fetoproteins zur Herstellung von Reagentien für einen Radioimmuntest zur Bestimmung von $\alpha_1$-Fetoproteinen, den Radioimmuntest selbst und einen entsprechenden Reagenzsatz.

EP 0 001 812 A1

Croydon Printing Company Ltd.

27. Oktober 1978
RAN 4093/36

F. Hoffmann-La Roche & Co. Aktiengesellschaft, Basel/Schweiz

# Verfahren zur Herstellung gereinigten $\alpha_1$-Fetoproteins und dessen Verwendung

Die Erfindung bezieht sich auf ein Verfahren zum Isolieren von $\alpha_1$-Fetoprotein, das ausreichend rein ist, um bei Radioimmuntests brauchbar zu sein, zu denen Arbeitsweisen und Techniken der Affinitätschromatographie, der Adsorptionschromatographie mit immobilisiertem reaktivem Farbstoff und Polyacrylamid-Gelelektrophorese gehören.

Ein Fetus-spezifisches Serumprotein wurde zuerst von Pederson, Nature (London) 154, 575 (1944) in Kalbserum und von Bergstrand et al., Scand. J. Clin. Lab. Invest 8, 174 (1956) beim Menschen nachgewiesen. Dieses Fetusspezifische Protein wurde aufgrund seiner elektrophoretischen Beweglichkeit als $\alpha_1$-Fetoprotein (AFP) bezeichnet.

$\alpha_1$-Fetoprotein in Seren hepatozellulären Krebses und von Patienten mit bösartigen Embryonal-Teratomen erwies sich als immunologisch und elektrophoretisch identisch mit $\alpha_1$-Fetoprotein, das in fetalem Serum von Tatarinov, Vopr. Med. Khim. 11,20-24, (1965) und Abelev et al., Int. J. Cancer 2, 551-558 (1967) gefunden wurde.

Human-$\alpha_1$-fetoprotein wurde aus Fetalserum nach chemischen Verfahren von Pederson, Clin. Chimica Acta 38, 163-170 (1971), Ruoslahit et al., Int. J. Cancer 7, 218-225 (1971), Silver et al., Proc. Nat. Acad. Sci., U.S.A. 70, 526-530 (1973), Forrester et al., Clin. Chimica Acta 64, 317-323 (1975) und Twomey et al., Clin. Chem. 22, 1306-1309 (1976) isoliert. Außerdem wurden immun-chemische und chemische Methoden angewandt, um $\alpha_1$-Fetoprotein aus Hepatom -Serum zu isolieren, und zwar von Nishi, Cancer Res. 30, 2507-2513 (1970), Nishi et al., Protides Biol. Fluids 18, 43-47 (1971), Nishi et al., Biochem. Biophys. Acta 278, 293-298 (1972), Aoyagi et al., Cancer Research 37, 3663-3667 (Okt. 1977), Yachnin et al., Biochem. Biophys. Acta 493, 418-428 (1977), Lehmann et al., Clin. Chimica Acta 33, 197-206 (1971) und Alpert et al., J. Biol. Chem. 247, 3492-3497 (1972).

Die zuvor erwähnten Methoden führen zu einem $\alpha_1$-Fetoprotein (AFP), das nicht rein ist, da es Albumin und andere Proteine enthält. Aufgrund des Vorhandenseins dieser Verunreinigungen sind solche verhältnismäßig unreinen $\alpha_1$-Fetoprotein-Präparate unbefriedigend als radioaktiv markiertes Antigen für einen empfindlichen Radioimmuntest. Ferner sind solche Präparate unbefriedigend für die Gewinnung monospezifischer Antiseren mit hohem Titer für einen empfindlichen Radioimmuntest (RIT). Da Proben, die auf AFP bei einem Radioimmuntest untersucht werden sollen, AFP häufig in geringer Konzentration enthalten, ist es wesentlich, daß das RIT-Verfahren äußerst empfindlich ist. Mit "äußerst

empfindlich" ist gemeint, daß der RIT in der Lage sein muß, AFP mit einem Gehalt von etwa 20 ng/ml der Probe genau nachzuweisen. Solche empfindlichen RIT-Arbeitsweisen werden zur Erfassung von Geburtsdefekten bei Schwangeren verlangt, was bislang nicht möglich war.

Nicht spezifische Antiseren oder unreines markiertes AFP können bei solchen empfindlichen RIT-Verfahren nicht angewandt werden, da sie kein genaues Maß für den AFP-Gehalt der Probe liefern würden. Zudem sind die in der Literatur beschriebenen Methoden zum Isolieren von AFP aus großen Volumina von Lösungen der Quelle und/oder solchen Lösungen der Quelle, die geringe AFP-Konzentrationen aufweisen, unbefriedigend. Schließlich scheinen die in der Literatur für die Isolierung von AFP offenbarten Methoden für Automatisierungstechniken unbefriedigend zu sein.

Insbesondere der Aufsatz von Ruoslahti et al. lehrt ein Verfahren zum Reinigen von AFP aus fetalem Serum unter Anwendung von zwei hintereinandergeschalteten Elektrofokussierverfahren oder von Immunfällung. Die Elektrofokussierverfahren beruhen auf ladungsbedingten Trennungen. Es gibt jedoch keinen Hinweis darauf, daß das durch die angegebene Arbeitsweise erhaltene AFP genügend rein ist, um bei einem RIT eingesetzt werden zu können.

Alpert et al. lehren ein Verfahren zum Reinigen von AFP nach einem dreistufigen chemischen Verfahren: (1) Stärkegel-Blockelektrophorese, (2) Gelfiltration unter Verwendung einer Sephadex-Säule und (3) isoelektrisches Fokussieren. Das so erhaltene AFP soll laut Immunodiffusion und Natriumdodecylsulfat-Gelelektrophorese rein sein. Außerdem soll das so erhaltene AFP in der Lage sein, immunospezifisches Antiserum in Kaninchen zu erzeugen. Im Gegensatz zu dem hier beanspruchten Verfahren, das sowohl immunologische als auch chemische Techniken anwendet,

findet sich dort kein Hinweis darauf, daß das nach dem offenbarten Verfahren gebildete AFP für RIT-Arbeitsweisen rein genug ist.

Die ersten beiden oben erwähnten Nishi-Veröffentlichungen verwenden Hepatom-Serum als Ausgangsmaterial und reinigen auf der Grundlage der Immunfällung mit anschließender Gelfiltration.

Auch das Verfahren der dritten Nishi-Veröffentlichung verwendet Hepatom-Serum als Ausgangsmaterial und lehrt eine Reinigungsmethode, die eine Immunosorbens-Säule anwendet. Der Antikörper wird jedoch nicht adsorbiert, um Monospezifität zu gewährleisten. Weitere Reinigung erfolgt durch Gelfiltration.

Keine der in den Nishi-Veröffentlichungen offenbarten Methoden scheint dem Fachmann die Lehre zu geben, wie AFP so zu reinigen ist, daß es für die Verwendung in RIT-Arbeitsweisen ausreicht. Auf jeden Fall läßt keine einzige auch nur erkennen, daß AFP aus von Affen stammendem Ausgangsmaterial in ausreichender Reinheit zur Verwendung bei Human-RIT-Verfahren erhalten werden kann.

Aoyagi et al. lehren ein Verfahren zur Reinigung von AFP aus Nabelschnur-Serum unter Verwendung einer Immunosorbens-Säule, Ionenaustausch an DEAE-Sephadex und Gelfiltration. Auch hier findet sich kein Hinweis darauf, daß das nach diesem Verfahren gereinigte AFP für RIT-Arbeitsweisen geeignet ist. Wenngleich Ionenaustausch und Gelfiltrations-Chromatographie keine optimale Trennung von Albumin bieten, da Ladung und Molekulargewicht zu nahe beieinanderliegen, scheint das nach dieser Arbeitsweise erzeugte AFP verhältnismäßig rein zu sein. Doch findet sich kein Hinweis, daß das offenbarte Verfahren zu einem AFP aus von Affen stammendem Ausgangsmaterial führen könnte, das für Human-RITs geeignet wäre, im Gegensatz zum erfindungsgemäßen Verfahren.

Yachnin et al. offenbaren ein Verfahren zum Reinigen von AFP-Hepatom-Serum unter Verwendung einer Immunosorbens-Säule und Anwendung der Gelfiltration. Die Beschreibung scheint erkennen zu lassen, daß das Produkt mikroheterogen und deshalb für RIT-Arbeitsweisen unbefriedigend ist.

So besteht ein Bedarf nach einem Verfahren zum Isolieren von $\alpha_1$-Fetoprotein so rein wie möglich, was Albumin und andere Protein-Verunreinigungen betrifft, die den empfindlichen Radioimmuntest stören würden. Ferner besteht ein Bedarf nach Isolierung von AFP, die auf große Volumina von Ausgangslösungen und/oder Lösungen des Quellenmaterials, die AFP in geringer Konzentration enthalten, wirksam angewandt werden kann. Schließlich besteht ein Bedarf nach einem Verfahren zum Isolieren von AFP, das ganz oder teilweise der Durchführung in automatischen Vorrichtungen und nach automatischen Techniken angepaßt werden kann. Dem kann insgesamt durch das erfindungsgemäße Verfahren genüge getan werden. Ferner führt die erfindungsgemäße Lehre zu Maßnahmen, durch die AFP aus Affen-Hepatomserum in für praktische Zwecke ausreichender Reinheit, gleichermassen wie aus menschlichem Nabelschnur-Serum und ausreichend rein, um bei RIT-Arbeitsweisen an menschlichen Proben verwendet zu werden, erhalten werden kann. AFP von Affen ist bislang nicht als bei Radioimmuntests an menschlichem Material brauchbar erkannt worden.

Die Erfindung führt zu einem Verfahren zum Isolieren von $\alpha_1$-Fetoprotein aus biologischem Ausgangsmaterial, wie Nabelschnurblut, Hepatomgewebe und dessen Extrakten, anderen

Tumoren, Bauchhöhlenflüssigkeit, fetalem Blut, Embryonalgeweben, Amnion-Flüssigkeit, Hepatomblut und in Kultur oder in Wirtstieren gezüchteten Zellen. Sie führt ferner zu einem Verfahren zur Reinigung von $\alpha_1$-Fetoprotein (AFP) aus Quellen von Affen, insbesondere Affen-Hepatomblut. Es hat sich gezeigt, daß aus dieser Quelle erfindungsgemäß gereinigtes AFP immunologisch identisch ist mit AFP, das in menschlichem Nabelschnur-Blut und menschlicher Bauchhöhlenflüssigkeit enthalten ist. Die Verwendung von Quellenmaterial aus Affen, wie Hepatomblut, zur Isolierung von reinem AFP nach dem erfindungsgemäßen Verfahren ist von Bedeutung, da ein solches Material einheitlicher und wesentlich reicher an AFP ist als bestimmte andere Quellen, wie menschliches Nabelschnur-Blut. Die AFP-Konzentration in Affen-Hepatomblut liegt im Bereich von 1 bis 5 mg/ml, im Gegensatz zu menschlichem Nabelschnur-Blut, das etwa 0,06 bis etwa 0,1 mg/ml enthält.

Das erfindungsgemäße Verfahren liefert ein reines $\alpha_1$-Fetoprotein (AFP), das im wesentlichen frei ist von Albumin und anderen Protein-Verunreinigungen und beim empfindlichen Radioimmuntest verwendet werden kann. Beim Verfahren sind folgende Techniken nacheinander beteiligt:

a) Affinitätschromatographie an einer Immunosorbens-Säule,
b) Adsorptionschromatographie eines immobilisierten reaktiven Farbstoffs,
c) gegebenenfalls Gelfiltration,
d) gegebenenfalls Mischionenaustausch-Cellulosechromatographie und
e) Polyacrylamid-Gelelektrophorese.

Wird bei den ersten beiden Maßnahmen ein bestimmtes Ausmaß an Herabsetzung der Art und/oder Menge der Verunreinigungen nicht erzielt, erfolgen Gelfiltration und

Mischionenaustausch-Cellulosechromatographie zur Vorbereitung der Polyacrylamid-Gelelektrophorese.

Insbesondere bezieht sich die Erfindung auf ein Verfahren zur Herstellung gereinigten $\alpha_1$-Fetoproteins mit folgenden Schritten:

a) Puffern einer Probe eines Materials aus einer biologischen Quelle, das dafür bekannt ist, $\alpha_1$-Fetoprotein zu enthalten, auf einen pH von etwa 7,
b) Affinitätschromatographie der gepufferten Probe durch Hindurchführen durch eine Säule mit einem für $\alpha_1$-Fetoprotein spezifischen Antikörper, der auf einem festen Träger unbeweglich verankert ist und Eluieren des gebundenen $\alpha_1$-Fetoproteins durch Behandeln der Säule mit einem geeigneten Desorptionsmittel,
c) Entfernen praktisch des gesamten im Eluat der Stufe b) vorhandenen Albumins mit Hilfe der Adsorptionschromatographie durch Hindurchleiten durch eine Säule, die einen immobilisierten reaktiven Farbstoffadsorber enthält,
d) Dialysieren der das nicht-adsorbierte $\alpha_1$-Fetoprotein enthaltenden Lösung der Stufe c),
e) Lyophilisieren der dialysierten Lösung der Stufe d),
f) Lösen des in Stufe e) gebildeten Pulvers in einem geeigneten Puffer und Polyacrylamid-Gelelektrophorese der erhaltenen Lösung.

Das anfallende Produkt, $\alpha_1$-Fetoprotein, ist homogen, d.h., es erscheint als einzelne Bande bei der analytischen Disk-Elektrophorese. Nach Immunoelektrophorese zeigt das nach dem erfindungsgemäßen Verfahren erhaltene AFP eine elektrophoretische Mobilität im Bereich des $\alpha_1$-Globulins, zeigt eine einzelne Präzipitin-Linie gegen antinormales Humanserum oder Antihuman-Serumalbumin mit hohem Titer. Diese Beobachtungen wurden durch Radioimmunoelektrophorese bestätigt. Erfindungsgemäß gereinigtes AFP zeigt eine Identitätslinie bei Doppel-Immunodiffusion mit $\alpha_1$-Fetoprotein in

Nabelschnur-Serum, hepatozellulärem Krebs-Serum und Bauchhölenflüssigkeit und ist praktisch frei von Albumin und anderen Proteinen, die seine Verwendung bei empfindlichen Radioimmuntests (RITs) stören würden.

Das erfindungsgemäße Verfahren umfaßt eine Kombination immunologischer, chemischer, Filtrations-, elektrostatischer und elektrophoretischer Methoden und Techniken.

Affinitätschromatographie

Um die Masse der Fremdproteine aus einer $\alpha_1$-Fetoprotein enthaltenden Probe der Lösung einer Quelle, wie menschlichem Nabelschnur-Serum oder Affen-Hepatomserum, zu entfernen, wird die Probe zunächst der Affinitätschromatographie unterworfen.

Die Affinitätschromatographie ist eine immunologische Adsorptionstechnik, bei der das Antigen aus der Lösung entfernt wird, indem es an ein Immunosorbens adsorbiert wird, das Antikörper umfaßt, die für das zu isolierende Antigen spezifisch sind, kovalent an einen festen, teilchenförmigen Träger gebunden. Proteine, die nicht mit dem immobilisierten Antikörper reagieren, werden nicht an den festen Träger gebunden. Diese ungebundenen Verunreinigungen werden daher leicht von dem gebundenen Antigen abgetrennt. Das Antigen wird danach von dem Antikörper abgetrennt, der an den festen Träger gebunden bleibt.

Um sicherzustellen, daß die Affinitätschromatographie für das gesuchte Antigen spezifisch ist, muß das Immunosorbens behandelt werden, um die nicht spezifischen Stellen zu blockieren, die übrigbleiben, nachdem der Antikörper daran gekuppelt ist. Dazu wird das durch Kuppeln des Antikörpers an einen festen Träger gebildete Immunosorbens zuerst mit einem geeigneten Puffer ins Gleichgewicht

gebracht. Das Immunosorbens wird dann nacheinander mit normalem Human-Serum, wieder mit dem gleichen Puffer der zur Gleichgewichtseinstellung verwendet wurde, und mit Ammoniumthiocyanat behandelt. Darauf wird das Immunosorbens wieder unter Verwendung des gleichen Puffers ins Gleichgewicht gebracht.

Nachdem das Immunosorbens ins Gleichgewicht gebracht ist, wird die Probe durch die Säule geführt und das Antigen, AFP, wird auf seinen Antikörper adsorbiert, während Fremdproteine eluiert werden. Die Probe wird anfangs mit Puffer vom pH 7,0 verdünnt, so daß eine theoretische AFP-Konzentration von etwa 0,05 bis etwa 0,1 mg/ml erhalten wird. Im Falle von z.B. Nabelschnur-Serum wird eine 1:1-Volumenverdünnung mit dem Puffer vorgenommen.

Nachdem die Probe durch die Säule geführt worden ist, wird das Antigen unter Verwendung eines Desorptionsmittels eluiert, das den Antigen/Antikörper-Komplex aufbricht. Geeignete Desorptionsmittel sind z.B. 6- bis 8-molare Lösungen von Harnstoff, wäßrige Natriumchloridlösungen mit hoher Konzentration, d.h. 0,5-molar und höher, saure Puffer, d.h. Puffer mit einem pH von etwa 2 bis etwa 3 und Puffer mit chaotropen Ionen, d.h. Ionen, die bekanntlich die Fähigkeit haben, Antigen/Antikörper-Bindungen aufzubrechen, wie z.B. das Thiocyanation. Ein bevorzugtes Desorptionsmittel ist 3,0 m Ammoniumthiocyanat in 0,1 m Natriumphosphat-Puffer bei pH 7,0. Die Desorption des AFP von der Chromatographie-Säule regeneriert diese, sie braucht zur Wiederverwendung nur die Gleichgewichtseinstellung mit einem geeigneten Puffer.

Die vereinigten Eluate der adsorbierten Fraktion, d.h., der AFP-haltigen Fraktion, werden dann gegen entionisiertes Wasser dialysiert und das erhaltene Dialysat durch Ultrafiltration aufkonzentriert. Die adsorbierte Fraktion

wird dann auf ihren AFP-Gehalt nach immunologischen Techniken getestet.

Die zur Verwendung als feste Träger für den Antikörper geeigneten Materialien müssen eine Reihe kritischer Parameter erfüllen, d.h., sie müssen

a) γ-Globulin (Antikörper) ohne Beeinträchtigung ihrer immunologischen Eigenschaften immobilisieren,
b) eine Probe einer Testflüssigkeit durch eine Säule mit annehmbarer Geschwindigkeit hindurchlassen,
c) inert sein, d.h., sie dürfen keine immunologisch störenden Eigenschaften haben oder chemisch oder immunologisch modifiziert werden können, damit sie keine solchen störenden Eigenschaften haben,
d) die Desorption von Antigenen zulassen, ohne daß der Antikörper desorbiert oder immunologisch beeinträchtigt wird, und
e) zur Verwendung in einem automatisierten System geeignet sein.

Materialien, die diese Eigenschaften haben, sind z.B. Agarosegele, poröse Glasperlen, Polyacrylamid-Perlen und dergleichen, vorzugsweise Agarosegele. Agarose ist der neutrale Anteil von Agar. Agarosegel ist im Handel erhältlich (z.B. unter der Bezeichnung "Sepharose"). Die im Handel erhältlichen Agarosegele sind wäßrige Suspensionen, die gewöhnlich ein Konservierungsmittel enthalten, z.B. 0,02 % Natriumazid. Das Gel wird in Perlenform mit einer ausgewählten Teilchengröße und einer bestimmten Prozentkonzentration Agarose hergestellt. Die Konzentration der Agarose in dem Gel bestimmt dessen Fraktionierungsbereich.

Die zur Verwendung bei der Affinitätschromatographie des erfindungsgemäßen Verfahrens am meisten geeigneten Gele, d.h., die alle oben genannten Kriterien erfüllen, sind im Handel erhältlich (unter der Bezeichnung "Sepharose 4B").

Diese Gele haben eine Teilchengröße von 40 bis 190 µm und enthalten 4 Gew.-% Agarose. Ferner haben diese Gele einen Fraktionierungsbereich von 3 x $10^5$ bis 3 x $10^6$.

Der bei der Affinitätschromatographiestufe des erfindungsgemäßen Verfahrens bevorzugt verwendete Puffer ist ein 0,1 m Phosphatpuffer bei pH 7,0. Weitere verwendbare Puffer sind z.B. Boratpuffer bei pH 8, Acetatpuffer bei einem pH von etwa 5 und dergleichen. Die Wahl eines bestimmten Puffers hängt von Faktoren wie der Stabilität des zu isolierenden Antigens und dergleichen ab.

Die bevorzugte Methode zum Konzentrieren des Dialysats durch Ultrafiltration besteht darin, eine gerührte Zelle mit einer Membran mit einer Molekulargewicht-Obergrenze von 10.000 zu verwenden, wie einer handelsüblichen PM-10-Membran (der Amicon Corp., Lexington, Massachusetts). Das Dialysat kann auch nach anderen, auf dem Fachgebiet bekannten Methoden konzentriert werden, wie z.B. durch Gefriertrocknen.

Der AFP-Gehalt des erhaltenen Materials kann nach mehreren immunologischen Testmaßnahmen erfasst werden, der Radioimmuntest (RIT) ist jedoch bevorzugt. Wo möglich, ermöglicht und sichert der Zusatz einer geringen, abgemessenen Menge von radioaktiv markiertem AFP zu der teilweise gereinigten Probe und das Erfassen des Durchgangs durch die verschiedenen Stufen des Verfahrens die Reinheit des fertigen Präparats.

Die Stufe der Affinitätschromatographie des erfindungsgemäßen Verfahrens kann automatisiert werden, indem eine modifizierte Version einer als "Cyclum" bezeichneten Vorrichtung verwendet wird (die vom Oak Ridge National Laboratory, Oak Ridge, Tennessee, entwickelt wurde). Die Cyclum-Schalttafel mit der zugehörigen Ausstattung

ist unter Verwendung eines Nockenzeitgebers programmiert, um eine von drei Flüssigkeiten wahlweise durch eine Affinitätssäule fließen zu lassen, die optische Dichte des abfließenden Materials zu erfassen und aufzuzeichnen und das abfließende Material in einen von drei Behältern zu leiten. Die Cyclum-Vorrichtung, die die Affinitätschromatographie-Behandlung kontinuierlich erneut durchzuführen vermag, ist von Anderson et al. in Cancer Research, Band 34, S. 2066 bis 2076 (1974) beschrieben.

Adsorptionschromatographie mit immobilisiertem Reaktivfarbstoff

Das in der bei der vorhergehenden Stufe angefallenen adsorbierten Fraktion vorhandene Albumin wird aus dem AFP mit Hilfe eines unbeweglich gemachten Reaktivfarbstoff-Adsorbens praktisch entfernt. Beispiele für geeignete Farbstoffe sind die sogenannten Reaktivfarbstoffe, z.B. der Cibacron-Reihe. Diese Farbstoffe leiten sich von Cyanursäurechlorid ab, d.h., 2,4,6-Trichlor-1,3,5-triazin. Die für die praktische Durchführung der Erfindung geeigneten Farbstoffe sind sulfonierte, polyaromatische Farbstoffe mit einem 1,3,5-Triazin-Kern und einem einzelnen reaktiven Chlor. Beispiele für geeignete Farbstoffe sind Cibacron Brilliant-Blau, Cibacron Scharlach und Cibacron Brilliant-Orange. Bevorzugt wird Cibacron Brilliant-Blau der Struktur

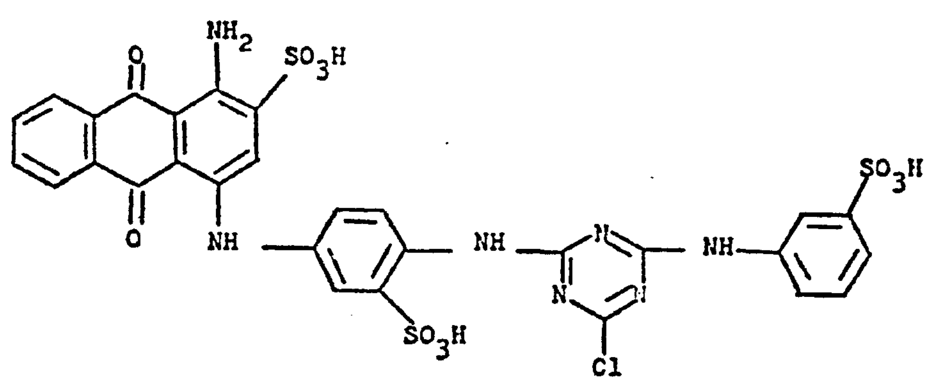

Der Reaktivfarbstoff wird immobil gemacht, indem er gebunden wird, d.h. kovalent gebunden oder adsorbiert an einen festen Träger, wie oben bezüglich der Affinitätschromatographie erörtert. Ein erfindungsgemäß bevorzugter immobilisierter Reaktivfarbstoff ist Sepharoseblau-Dextran. Dieser Farbstoff wird durch kovalentes Binden von Blaudextran, d.h. Dextran mit chemisch daran gebundenem Cibacron Brilliant-Blau, an Agarosegel, z.B. Sepharose 4B, hergestellt. Das Binden des blauen Dextrans an Sepharose erfolgt nach der Methode von Nishikawa et al., Anal. Biochemistry, Band 64, Seiten 268-275 (1975). Zu diesem Verfahren gehört die Aktivierung der Sepharose mit auf pH 11 gepuffertem Bromcyanid bei etwa 4°C. Das blaue Dextran wird dann in 0,1 m Phosphatpuffer bei pH 7 zugesetzt und die Reaktion 24 h bei etwa 4°C ablaufen gelassen. Die aktivierte Sepharose wird an den Farbstoff über eine der aromatischen Gruppen gebunden. Das fertige Präparat vermag 10 mg Albumin/ml Gelpackung zu binden.

Die Adsorptionschromatographie mit immobilisiertem Reaktivfarbstoff erfolgt unter Verwendung einer Säule von 5 x 13 cm. Beispielsweise kann die von Travis et al., in Clin. Chimica Acta 49, 49 bis 52 (1973) beschriebene Arbeitsweise angewandt werden, um das Albumin selektiv aus den AFP-haltigen Fraktionen zu entfernen. So werden die vereinigten AFP-haltigen Fraktionen aus der Affinitätssäule gegen den Säulenpuffer dialysiert, d.h. gegen 0,05 m Tris - 0,5 m NaCl, pH 8,0, bevor sie durch das Bett aus immobil gemachtem Reaktivfarbstoff-Adsorbens gepumpt werden. Das nicht-adsorbierte Eluat, das AFP enthält, wird zusammengefasst und durch Ultrafiltration oder nach anderen geeigneten Maßnahmen, wie in der vorangehenden Stufe, aufkonzentriert. Das anfallende AFP-haltige Material ist praktisch frei von Albumin, wie durch

immunologische Techniken bestimmt wurde. Etwa 90 bis 95 % des in der Probe ursprünglich vorhandenen Albumins werden durch diese Technik entfernt. Die Adsorptionschromatographie-Säule mit immobilisiertem Reaktivfarbstoff kann durch Behandeln mit einer 6,0-molaren Harnstofflösung zum Entfernen des Albumins regeneriert werden, worauf sich eine Gleichgewichtseinstellung mit einem geeigneten Puffer anschließt.

Gelfiltrationschromatographie

Die nicht-adsorbierte Fraktion aus der Adsorptionschromatographie-Säule mit immobilgemachtem Reaktivfarbstoff, die AFP enthält, kann dann der Gelfitrationschromatographie unterworfen werden, um die hochmolekularen Verunreinigungen und einen Teil irgendwelchen restlichen Albumins abzutrennen. Die Molekulargewichte von AFP und Albumin sind 72.000 bzw. 69.000 Daltons. Unter hochmolekularen Verunreinigungen werden Proteine mit einem Molekulargewicht von etwa 100.000 oder darüber verstanden. Die bevorzugte Säule für diese Behandlung enthält ein Filtermaterial, das ein hydrophiles, wasserunlösliches, vernetztes Dextranpolymergel ist. Dieses Material und das Verfahren zu seiner Herstellung sind in der GB-PS 854 715 beschrieben. Das bevorzugte Gelmaterial, das im Handel (von der AB Pharmacia, Uppsala, Schweden, unter der Bezeichnung "Sephadex") erhältlich ist, weist ein dreidimensionales makroskopisches Gitter von miteinander gebundenen oder vernetzten Dextransubstanzen auf, das Wasser unter Quellung zu adsorbieren vermag. Die Fähigkeit des Gelmaterials zur Wasseraufnahme ist umgekehrt proportional zum Vernetzungsgrad der Dextransubstanzen im Gelmaterial. Das Gelmaterial steht in einer Anzahl verschiedener Qualitäten zur Verfügung, die sich hinsichtlich des Porositätsgrades unterscheiden. Das für die erfindungsgemäße Verwendung bevorzugte Gel hat einen ungefähren Molekulargewichts-

Ausschlußgrenzwert von 200.000, eine Wasserwiederaufnahme (g $H_2O$/g Trockengel) von 20 $\pm$ 2,0, eine Teilchengröße von 40 bis 120 µm und ein Bettvolumen/ml/g Trockengel von 30 bis 40. Dieses besondere Gel wird als "Sephadex G-200" bezeichnet.

Die Gelsäule wird mit 0,05 m monobasischem Natriumphosphat-0,15 m Natriumchlorid-Puffer, pH 5,0, ins Gleichgewicht gebracht. Die nicht-adsorbierte Fraktion aus der Adsorptionschromatographie-Säule mit immobilgemachtem Reaktivfarbstoff, die AFP enthält, wird aufkonzentriert und durch diskontinuierliche Diafiltration gegen den gleichen Puffer dialysiert und dann an der im Gleichgewicht befindlichen Gelsäule aufströmend chromatographiert. Aufsteigender Strom wird angewandt, da er die Abtrennung des AFP von niedrig- und hochmolekularen Proteinverunreinigungen erleichtert und die Integrität des Gelbettes erhält.

Das Eluat aus der Säule wird in Fraktionen von z.B. 10 ml aufgefangen. Diese Fraktionen werden auf ihren Proteingehalt und ihre Radioaktivität hin erfasst. Außerdem wird der AFP-Gehalt bestimmter Fraktionen durch RIT bestätigt. Im allgemeinen werden etwa 150 solcher Fraktionen erhalten. Die Fraktionen mit dem höchsten AFP-Gehalt werden aufgefangen und vereinigt. Der Rest wird verworfen. Im allgemeinen wurde beim Auffangen von 150 Fraktionen festgestellt, daß die Fraktionen 100 bis 120 AFP maximal enthielten.

Ionenaustausch-Säulenchromatographie an Cellulose

Um noch mehr verunreinigende Proteine aus den AFP enthaltenden Fraktionen aus der Stufe der Gelfiltrationschromatographie zu entfernen, können die vereinigten Fraktionen der Ionenaustausch-Säulenchromatographie an Cellulose unterworfen werden. Diese Maßnahme trennt die Proteine

aufgrund ihres elektrostatischen Bindungsvermögens im Gegensatz z.B. zur vorher durchgeführten Gelfiltrationschromatographie ab, die die Proteine nach ihrer Größe abtrennte.

Die zur Verwendung bei dieser Arbeitsweise als geeignet festgestellte Ionenaustauscher-Säule ist eine Mischbett-Säule aus einem kationischen Austauscher, z.B. Carboxymethylcellulose, und einem Anionenaustauscher, z.B. Diäthylaminoäthylcellulose.

Die für diese Arbeitsweise geeigneten Carboxymethylcellulosen sind z.B. solche in mikrokörniger Form, mit stäbchenförmigen Teilchen einer Teilchengrößenverteilung, ausgedrückt als Durchmesser gleichwertiger Kügelchen, im Bereich von etwa 20 bis 60 µm mit einer Kapazität von 1,0 $\pm$ 0,1 mÄq/g und einem Wasser-Wiederaufnahmevermögen von 2,3 bis 2,7 g/g trockenem Austauscher. Die bevorzugte Ionenform ist die $Na^+$-Form. Ein geeigneter Ionenaustauscher ist in trockener Form im Handel erhältlich (unter der Bezeichnung "CM 52" der H. Reeve Angel Inc., Clifton, New Jersey).

Die für die Verwendung bei dieser Arbeitsweise am meisten geeigneten Diäthylaminoäthylcellulose-Präparate sind solche, die in mikrokörniger Form vorliegen, stäbchenförmige Teilchen mit einer Teilchengrößenverteilung, ausgedrückt als Durchmesser gleichwertiger Kügelchen, im Bereich von etwa 20 bis 60 µm, eine Kapazität von 1,0 $\pm$ 0,1 mÄq/g, ein Wasser-Wiederaufnahmevermögen von 2,3 bis 2,8 g/g trockenem Austauscher haben und in freier Basenform vorliegen. Ein geeigneter Ionenaustauscher ist z.B. ein (im Handel unter der Bezeichnung "DE 52" der H. Reeve Angel Inc., Clifton, New Jersey, erhälticher) Austauscher.

Die Misch-Ionenaustausch-Cellulosesäule wird wie folgt hergestellt: Zuerst werden die Feinteile aus jedem Austauscher entfernt, z.B. durch Absaugen der überstehenden Flüssigkeit, erhalten durch Zugabe eines zehnfachen Volumens Wasser, Rühren und Absitzenlassen der Feststoffe. Dann wird jeder Austauscher getrennt mit einer 2-molaren Natriumchloridlösung in 0,1 m Ammoniumacetat ins Gleichgewicht gebracht. Nach dem Absaugen der überstehenden Flüssigkeit wird eine Pufferlösung von 0,1 m Ammoniumacetat zu jeder der Cellulosen gegeben, und gleiche Volumina einer jeden solchen Aufschlämmung werden vereinigt und unter Eigengewicht in eine Säule von 2,5 x 13,0 cm gepackt. Die erhaltene Säule wird mit dem zu verwendenden Puffer, d.h., 0,1 m Ammoniumacetat, ins Gleichgewicht gebracht.

Die vereinigten Fraktionen aus der Gelfiltration werden konzentriert und durch Dialyse gegen die 0,1 m Ammoniumacetat-Pufferlösung ins Gleichgewicht gebracht. Die AFP-haltige Lösung wird dann auf die Ionenaustauscher-Säule mit 60 ml/h aufgebracht.

Nach dem Aufbringen der AFP-Lösung auf die Säule wird diese mit zusätzlichem Puffer gewaschen, um alles Material, das sich nicht daran bindet, zu entfernen. Danach wird ein linearer Natriumchlorid-Gradient durch sehr allmähliche Zugabe von Natriumchlorid zu dem Puffer eingestellt. Mit zunehmender Salzkonzentration im Puffer werden 3 ml-Fraktionen aufgefangen und, wie bei der vorhergehenden Stufe, auf Proteingehalt, Radioaktivität und auf AFP nach dem RIT untersucht. Wiederum werden die AFP enthaltenden Fraktionen aufgefangen und vereinigt.

Präparative Polyacrylamid-Elektrophorese

Die Reinigung des AFP wird durch Polyacrylamid-Elektrophorese abgeschlossen. Diese Arbeitsweise, die AFP aus

verunreinigenden Proteinen auf der Grundlage der Unterschiede in der molekularen Ladung, Größe und Form trennt, entfernt alle verbliebenen störenden Spuren an Albumin, die vorhanden sein könnten. Diese Arbeitsweise erfolgt vorzugsweise unter Verwendung eines Buchler-Polyslab (Buchler Instruments, Fort Lee, New Jersey), einer vertikalen Platte von 170 x 3,0 mm aus einem auflösenden 7 %-Polyacrylamid-Gel mit einem auf das auflösende Gel aufpolymerisierten konzentrierenden Gel von 10 x 3,0 mm.

Der Unterschied zwischen dem konzentrierenden Gel und dem auflösenden Gel, wie oben beschrieben, beruht hauptsächlich auf der Porengröße, die bei ersterem größer ist. Die Varianz der Porengröße wird durch Variieren der relativen Anteile an Monomeren erreicht, d.h. Acrylamid und Comonomer (Vernetzer), d.i. N,N'-Methylenbisacrylamid im Polymerisationsgemisch. Zur Beschreibung dieser Gele, ihrer Herstellung und Verwendung bei den elektrophoretischen Arbeitsweisen vgl. Davis, "Disc Electrophoresis - II, Method and Application to Human Serum Proteins", Annal. N.Y. Acad. Sci., Band 121, S. 404 bis 427 (1964).

Die aus der vorangehenden Arbeitsweise aufgefangenen, vereinigten Fraktionen werden gegen Wasser dialysiert. Das Dialysat wird dann zu einem Pulver lyophilisiert. Das Pulver wird dann in einer geringen Menge eines als Elektrophorese-Puffer bezeichneten Puffers gelöst. Diese Pufferlösung enthält etwa 0,6 Gew.-% Tris, d.h. 2-Amino-2-(hydroxymethyl)-1,3-propandiol, und etwa 3 Gew.-% Glycin bei einem pH von etwa 8,3. Dann werden Saccharose und eine Spur eines geeigneten Farbstoffs, wie z.B. Bromphenolblau, der Probe zugesetzt. Das Bromphenolblau wird als Indikator für das Fortschreiten der Prozedur verwendet, da es vor den Proteinen durch die Gele wandert. Die Saccharose ist vorhanden, um die Dichte der Probenlösung

etwas zu erhöhen. Vorzugsweise wird genügend Saccharose zugesetzt, um eine Konzentration von etwa 8 Gew.-% zu erzielen. Die AFP enthaltende Lösung wird in die Buchler-Polyslab-Platte gebracht und die Elektrophorese 24 h bei konstantem Strom von 40 mA durchgeführt. Während der Elektrophorese bilden die Proteine in der Schicht der Probenlösung, die 4 bis 6 mm hoch ist, zuerst eine dünne Bande, d.h., von etwa 1 mm Höhe, nahe dem Boden des konzentrierenden Gels, bevor sie durch die auflösende Gelschicht wandern.

Ist die Elektrophorese abgeschlossen, wird das auflösende Gel entfernt und quer zu dem Weg zwischen Kathode und Anode in 0,5 cm dicke Streifen geschnitten. Die Streifen werden einzeln mit 0,05 m Boratpuffer bei pH 8,4 eluiert. Die Eluate der Streifen werden durch Radioaktivität, Doppel-Immundiffusion und RIT auf ihren AFP-Gehalt geprüft.

Das vorstehend beschriebene fünfstufige Verfahren ist erfindungsgemäß erforderlich, um reines AFP aus bestimmten Quellen, wie menschlichem Nabelschnur-Serum, zu erhalten. Es liegt jedoch im Rahmen der Erfindung, reines AFP auch von anderen Quellen zu erhalten, insbesondere aus Affen-Hepatomserum unter Anwendung von nur drei der fünf beschriebenen Stufen. Die Wahl zwischen diesen drei Stufen, d.h. der Affinitätschromatographie, der Adsorptionschromatographie mit immobilisiertem Reaktivfarbstoff und der Polyacrylamid-Elektrophorese, und dem zuvor beschriebenen fünfstufigen Verfahren erfolgt unter Berücksichtigung folgender Aspekte:

Die Wahl zwischen dem dreistufigen und dem fünfstufigen Verfahren zur Erzielung von reinem AFP gemäß der Erfindung erfolgt unter Berücksichtigung der Art und/oder der Menge der Verunreinigungen. Die Entscheidung, ob drei oder fünf Stufen erforderlich sind, wird getroffen, nachdem die

Chromatographie mit dem immobilgemachten Reaktivfarbstoff durchgeführt worden ist. Eine Probe der vereinigten, nicht adsorbierten Fraktionen aus der Chromatographie mit unbeweglich gemachtem Reaktivfarbstoff wird der analytischen Disk-Elektrophorese unterworfen. Zeigt diese Maßnahme Verunreinigungen von 20 % und mehr an, wird die fünfstufige Arbeitsweise angewandt. Aber selbst wenn der Gehalt an Verunreinigungen innerhalb annehmbarer Grenzwerte liegt, d.h. unter 20 %, wird dennoch eine fünfstufige Arbeitsweise angewandt, wenn die Verunreinigungen eine elektrophoretische Mobilität ähnlich der des AFP zeigen.

Wenn nur eine dreistufige Reinigung erforderlich ist, werden die vereinigten,nicht adsorbierten Fraktionen aus der Chromatographie mit dem immobilgemachten Reaktivfarbstoff dialysiert und lyophilisiert. Das erhaltene Pulver wird dann in dem Elektrophorese-Puffer gelöst und, wie oben beschrieben, der Polyacrylamid-Elektrophorese unterworfen.

Das vorstehend beschriebene Verfahren liefert unabhängig davon, ob drei oder fünf Stufen angewandt werden, reines AFP nach dem Ergebnis der analytischen Disk-Elektrophorese. Das z.B. aus Nabelschnur-Serum, Bauchhöhlenflüssigkeit und Hepatom-Serum erhaltene reine AFP zeigt bei der Immundiffusion immunologische Identität. Aus Affen-Hepatomserum erhaltenes AFP zeigt ebenso immunologische Idenität mit den aus menschlichen Quellen erhaltenen Präparaten. Die Beobachtung einer einzigen Präzipitinlinie bei der Immundiffusion für diese Präparate von gereinigten AFP gegen ein heterospezifisches, nicht adsorbiertes Kaninchen-Anti-AFP-Serum zeigte immunologische Homogenität. Die Reinheit des erfindungsgemäß erhaltenen AFP wurde auch durch Immunoelektrophorese des gereinigten AFP gegen Anti-AFP-Serum (eine Präzipitinlinie) und antinormales Humanserum-Anti-Humanserumalbumin mit hohem Titer (keine Präzipitinlinie) ermittelt.

Außer durch die zuvor erwähnten Methoden wurde die Reinheit des erfindungsgemäß erhaltenen AFP durch die spezifische Aktivität ermittelt. Die spezifische Aktivität ist gleich der Antikörperneutralisation (RIT), dividiert durch die Proteinkonzentration, und ist beschrieben von Lowry et al., J. Biol. Chem., Band 193, Seiten 265 bis 275 (1951). Rinderserumalbumin (Pentex, Cat. Nr. 83 - 301) wurde als Protein-Bezugsstandard verwendet. Die spezifische Aktivität des erfindungsgemäß hergestellten gereinigten AFP lag, wie gefunden wurde, im Bereich zwischen 0,87 und 0,95 für Nabelschnur-Serum-AFP und zwischen 0,94 und 0,99 für hepatozelluläres Karzinomserum-AFP. Im Gegensatz dazu lag die spezifische Aktivität von AFP-haltigen, vereinigten Fraktionen vor der präparativen Polyacrylamid-Elektrophorese im Bereich zwischen 0,45 und etwa 0,75 und lieferte ein radioaktiv markiertes Produkt, das bei der RIT-Arbeitsweise aufgrund großer Mengen jodierter, hoch- und niedermolekularer Verunreinigungen nicht verwendbar war.

Das erfindungsgemäß hergestellte, gereinigte AFP eignet sich zur Verwendung in einem hochempfindlichen diagnostischen Radioimmuntest auf hepatozellulären Krebs und Geburtsanomalien. Das bislang auf dem Fachgebiet bekannte AFP eignet sich nicht gut für eine solche Radioimmuntest-Arbeitsweise aufgrund der großen Menge an vorhandenem Albumin. Außer im Hinblick auf die Erleichterung einer erheblichen Entfernung von Albumin, das die empfindlichen RIT-Arbeitsweisen stört, ist das erfindungsgemäße Verfahren im Vergleich zu anderen Versuchen zur Reinigung von AFP, wie sie auf dem Fachgebiet bekannt sind, insoweit von Vorteil, als es AFP aus Lösungen wirksam isoliert, die es in geringer Konzentration enthalten, und außerdem insofern, als es durch Automation bei großen Volumina solcher Lösungen effektiv ist. Ferner liefert das erfindungsgemäße Verfahren aus Affen-Hepatomserum isoliertes AFP in

solcher Reinheit, daß es einem AFP, das aus menschlichen Quellen erhalten wurde, durch Aminosäureanalyse sehr ähnlich ist und anstelle von AFP aus menschlichen Quellen bei RIT-Arbeitsweisen verwendbar ist.

Zur Verwendung bei Radioimmuntest-Arbeitsweisen wird AFP, insbesondere Affen-AFP, mit radioaktiven Atomen radioaktiv markiert, die mit den chemisch reaktiven Gruppen reagieren und die Antigenizität nicht wesentlich herabsetzen. $^{125}I$ hat sich als besonders geeignet erwiesen. AFP kann nach auf dem Fachgebiet bekannten Methoden mit Radiojod markiert werden, wobei nur geringfügige Abwandlungen von Konzentration und Volumen erfolgen. Die Chloramin T-Methode von Hunter und Greenwood, beschrieben in J. Biochem. 91, 46 (1964) unter Verwendung von $^{125}I$ ist besonders brauchbar.

Die Umsetzung erfolgt z.B. durch Mischen von 50 µg AFP in Boratpuffer mit 5mCi $Na^{125}I$ (pH 8-11) und 100 µg frischem Chloramin T (Natrium-p-toluolsulfochloramin), alles in Boratpuffer. Die Reaktion erfolgt bei Raumtemperatur in 1 min und wird durch Zusatz von Natriummetabisulfit gestoppt. Das markierte Produkt wird mit 1 ml einer 5%igen Humanserumalbuminlösung gemischt und das Gemisch auf eine vernetzte Dextrangelsäule gebracht, z.B. Sephadex G-100, um das Produkt vom nicht-umgesetzten $^{125}I$ zu trennen. Das Produkt wird dann aus der Säule z.B. mit 0,1 m Tris-0,15 m Natriumchloridpuffer, pH 7, eluiert und in Röhrchen (5 ml-Fraktionen) mit 0,5 ml einer 5%igen Humanalbuminlösung aufgefangen. Das Produkt wird mit Boratpuffer mit 0,25 % Humanalbumin auf eine geeignete Konzentration verdünnt.

Das mit $^{125}I$, wie oben beschrieben, markierte Affen-AFP eignet sich besonders für die Radioimmuntest-Arbeitsweise.

Der Fachmann würde nicht annehmen, daß dieses Material offensichtlich so verwendet werden könnte, wenn berücksichtigt wird, daß bislang AFP nicht ausreichend rein für RIT-Arbeitsweisen hergestellt worden ist und ferner, daß die herrschende Meinung unter den Fachleuten war, daß nur Materialien menschlichen Ursprungs als markiertes Quellenmaterial für die quantitative Probe auf Human-AFP durch RIT verwendet werden könnte. Erfindungsgemäß gereinigtes AFP kann in einem hochempfindlichen Radioimmuntest auf AFP verwendet werden, d.h. bei einer Empfindlichkeit von etwa 0,5 ng bis etwa 18 µg.

Ein bevorzugter RIT unter Verwendung von erfindungsgemäß gereinigtem AFP ist eine Festphasenarbeitsweise mit immobilgemachtem Zweitantikörper. Bei dieser Arbeitsweise ist Kaninchen-Anti-AFP der erste Antikörper und Ziegenantiserum, d.h. Ziegen-Anti-Kaninchen-γ-Globulin, der zweite Antikörper. Das Standardmaterial für den Test ist teilweise gereinigtes Nabelschnurblut-AFP.

Bei der Durchführung des Radioimmuntests wird eine Standard-Konkurrenzhemmkurve aufgestellt. Sie ist ein Maß für die Komplexbildung zwischen dem zugesetzten Antigen mit spezifischen Antikörpern. Die Kurve gibt die AFP-Menge pro Einheit der Probe wieder. Die Messung erfolgt in ng pro Dosis pro Teilmenge der Standard-AFP-Lösung, aufgetragen gegen die Menge an mit radioaktiv markiertem reinem AFP komplex gebundenem Antikörper. Die erhaltene Kurve wird zur Bestimmung der AFP-Menge in einer Probe, d.h. Serum, Plasma oder Amnion-Flüssigkeit, verwendet.

Bei einem bevorzugten Verfahren wird ein gemessenes Standardmaterial in eine Reihe von Röhrchen gegeben, die Äthylendiamintetraessigsäure (ÄDTA)-Puffer, pH etwa 6, enthalten, das etwa 1 Vol.-% normales Ziegenserum enthält.

Eine abgemessene Menge des ersten oder Primär-Antikörpers, d.h. Kaninchen-Anti-AFP, verdünnt mit einem geeigneten Puffer, z.B. 0,05 m Boratpuffer, pH 8,4, mit 0,25 Gew.-% Humanalbumin wird jedem Röhrchen zugefügt.

Nach Zugabe von verdünntem Antikörper wird eine abgemessene Menge an radioaktiv jodiertem, reinem Affen-AFP in jedes Röhrchen gegeben. Die Zusatzmenge kann z.B. zwischen 0,6 und 1,0 ng liegen. Die erhaltenen Lösungen werden bei Raumtemperatur ausreichend lange bis zum Abschluß der Reaktion, gewöhnlich etwa 5 h, inkubiert. Ist die Inkubation abgeschlossen, wird der immobilgemachte zweite oder Sekundär-Antikörper, d.h. Ziegen-Antikaninchen-γ-Globulin, jedem Röhrchen zugesetzt und die Lösung wieder etwa weitere 5 min bei Raumtemperatur inkubiert. Der immobilisierte Antikörper komplexiert mit dem AFP/Primärantikörper-Komplex und ermöglicht somit seine Entfernung aus der Lösung.

Unter den vorstehend beschriebenen Bedingungen bleibt freies AFP in Lösung. Der $^{125}I$-Gehalt des Präzipitats oder der überstehenden Flüssigkeit wird dann mit einem geeigneten Zähler bestimmt, und die Radioaktivität kann dann gegen die Antigenkonzentration aufgetragen werden. So wird eine Kurve erhalten, die zur Bestimmung der AFP-Menge in einer Probe von Plasma, Serum oder Amnion-Flüssigkeit nach dem oben beschriebenen Verfahren verwendet werden kann.

Der zweite oder Sekundärantikörper kann nach einer Reihe von auf dem Fachgebiet bekannten Maßnahmen unbeweglich gemacht werden, z.B. nach den zuvor unter Bezugnahme auf das Immunosorbens beschriebenen Verfahren. Außer Perlen sind auch Glas- oder Polycarbonatstäbchen geeignet, den zweiten Antikörper unbeweglich zu machen. Eine bevorzugte Methode zum Immobilisieren des Sekundärantikörpers besteht darin, ihn an ein ungesintertes Fluorkohlenstoff-Polymeri-

sat nach der von Fishman, US-PS 3 843 443, beschriebenen Methode zu adsorbieren. Ein zum Immobilisieren des Sekundärantikörpers besonders bevorzugtes Fluorkohlenstoff-Polymerisat ist ungesintertes Polyvinylidenfluorid.

Natürlich ist die Erfindung, soweit sie sich auf Verbesserungen von RIT-Arbeitsweisen bezieht, nicht auf die oben beschriebene Arbeitsweise beschränkt. Jede auf dem Fachgebiet für AFP anerkannte Radioimmuntest-Arbeitsweise auf der Grundlage der Bildung eines Komplexes des AFP mit einem Antiserum in einer Analysenprobe, der Zugabe eines Reagenzes, d.h., von radioaktiv markiertem AFP, des Entfernens des erhaltenen Komplexes aus der Lösung und der Messung der Menge an aufgenommenem, radioaktiv markiertem Material gegen einen Standard kann unter Verwendung des erfindungsgemäß hergestellten, gereinigten AFP als radioaktiv markiertes Material verbessert werden.

Die Genauigkeit des RIT hängt von der Reinheit des radioaktiv markierten Materials ab. Da AFP-Radioimmuntests, wie sie vorstehend beschrieben sind, unter Verwendung von erfindungsgemäß gereinigtem AFP noch empfindlicher werden, können sie nun beispielsweise in einem Programm zur Erfassung von Geburtsfehlern bei schwangeren Frauen angewandt werden. Die Anwendung einer RIT-Arbeitsweise in einem solchen Programm ist bislang nicht für durchführbar gehalten worden.

Die folgenden Beispiele veranschaulichen die Erfindung noch weiter.

Beispiel 1

Gesammeltes menschliches Nabelschnurblut-Serum wurde von Vollschwangeren erhalten und bei -20°C gelangert. Das Serum

wurde aufgetaut, mit 100.000 UpM 1 h bei 4°C zentrifugiert und die überstehende Flüssigkeit mit einem gleichen Volumen an 0,1 m Natriumphosphatpuffer mit 0,02 Gew.-% Natriumazid, pH 7,0, verdünnt.

Affinitätschromatographie

15 ml-Portionen des wie oben hergestellten verdünnten Nabelschnur-Serums wurden durch ein automatisch rückführendes Chromatographiesystem geführt, das auf die Abgabe von drei Flüssigkeiten, d.h. Probe, Puffer und Desorptionsmittel in eine Affinitätssäule sowie auf getrenntes Auffangen von Abfall, nicht adsorbierter Fraktion und adsorbierter Fraktion programmiert war. Das Verfahren wurde kontinuierlich wiederholt, bis das verdünnte Nabelschnur-Serum durch die Säule chromatographiert worden war. Die adsorbierten Fraktionen wurden aufgefangen und gegen entionisiertes Wasser dialysiert, indem sie durch zwei Bio-Rad-Hohlfaser-Einheiten (Bio-Rad, Cat.# B/HDG-1), hintereinander geschaltet, geführt wurden. Das Dialysat wurde vereinigt und durch Ultrafiltration auf ein Volumen von etwa 20 ml konzentriert.

Die Säule des automatisierten Chromatographiesystems war mit einem Immunosorbens gepackt, das wie folgt hergestellt wurde:

$\alpha_1$-Fetoprotein wurde aus dem Serum eines Affen mit einem chemisch induzierten Hepatom isoliert. Das AFP wurde durch Affinitätschromatographie und aufsteigende Gelfiltrationssäulenchromatographie gereinigt und mit 0,15 m einbasischem Natriumphosphat - 0,15 m Natriumchlorid-Puffer vom pH 5,0 ins Gleichgewicht gebracht. Weiße Neuseeland-Kaninchen wurden mit dem Antigen immunisiert, das an methyliertes Rinderserumalbumin gekoppelt worden war, bevor es mit fertigem Freund'schem Adjuvans gemischt wurde. Die Bildung von Anti-AFP-Serum mit hohem Titer wurde

doppelt durch Immunodiffusion und Immunoelektrophorese überwacht.

Das so gebildete Kaninchen-Anti-AFP-(Affen)Serum wurde mit 3 mg/ml gepulvertem Human-Serumalbumin 1 h bei 37°C und dann 18 h bei 4°C adsorbiert und anschließend 1 h bei 100.000 UpM zentrifugiert. Die anfallenden 30 ml adsorbierten Antikörpers wurden der absteigenden DEAE-Cellulose-Säulenchromatographie an einer 2,5 x 40,0 cm-Säule in 0,01 m Natriumphosphatpuffer bei pH 8,0 unterworfen. Die Antikörper enthaltenden Fraktionen wurden vereinigt und kovalent an 50 ml Sepharose 4B, mit in N-Methyl-2-pyrrolidon gelöstem Bromcyanid aktiviert, gebunden. Die γ-Globulinfraktion wurde der aktivierten Sepharose 4B zugesetzt und die Reaktion 24 h bei 4° C ablaufen gelassen. Nach dem Mischen mit einem gleichen Volumen wäßrigen 1 m Äthanolamins für 1 h bei pH 8,0 wurde das Immunosorbens in 0,1 m Natriumphosphatpuffer bei pH 7,0 ins Gleichgewicht gebracht und in eine 5,0 x 30,0 cm messende Chromatographiesäule gebracht, die bis zu einer Höhe von 6 cm mit verstellbaren Strömungsregulatoren ausgestattet war. Um nicht-spezifische Adsorption von Protein zu verhindern, wurde das Immunosorbens durch aufeinanderfolgende Zugabe von 20 ml Normalserum, 0,1 m Phosphatpuffer vom pH 7,0, 3,0 m Ammoniumthiocyanat in 0,1 m Phosphatpuffer vom pH 7,0, behandelt und schließlich mit 0,1 m Phosphatpuffer vom pH 7,0 wieder ins Gleichgewicht gebracht.

Chromatographie mit immobilisiertem Reaktivfarbstoff

Die Adsorptionschromatographie mit immobilisiertem Reaktivfarbstoff wurde unter Verwendung von Sepharose-Blaudextran als immobilisiertem Reaktivfarbstoff durchgeführt. Sepharose-Blaudextran wurde durch kovalentes Binden von Blaudextran 2000 an Sepharose 4B hergestellt. Das Produkt wurde in eine 5 x 13 cm Säule gepackt. Die Probe aus der

Affinitätschromatographie wurde durch die Säule mit 120 ml/h gepumpt, um den größten Teil des vorhandenen Albumins zu entfernen. Fraktionen von 10 ml wurden aufgefangen. Das Gelbett wurde von nicht-adsorbiertem Protein freigewaschen. Eine 6 m Harnstofflösung im Säulenpuffer, d.h. 0,05 m Tris, also 2-Amino-2-(hydroxymethyl)-1,3-propandiol, und 0,05 m Natriumchlorid wurden zum Desorbieren des Albumins von der Säule zum Regenerieren verwendet. Die AFP-haltigen Fraktionen des nicht-adsorbierten Eluats wurden vereinigt und durch Ultrafiltration konzentriert. Um völlige Albumin-Entfernung zu gewährleisten, wurde die nicht-adsorbierte Fraktion erneut an der Säule chromatographiert. Die Säule wurde wieder, wie zuvor beschrieben, regeneriert.

In dieser und den folgenden Stufen wurde die Reinigung von AFP durch analytische Disk-Elektrophorese und immunologische Techniken, z.B. Immunoelektrophorese, Doppelimmunodiffusion und RIT, überwacht.

Gelfiltrationschromatographie

Die nicht-adsorbierte Fraktion aus der Sepharose-Blaudextran-Säule wurde konzentriert und auf ein Volumen von etwa 20 ml durch diskontinuierliche Diafiltration gegen 0,05 m monobasisches Natriumphosphat - 0,15 m Natriumchlorid-Puffer, pH 5,0, dialysiert. Die dialysierte Probe wurde zur Entfernung hochmolekularer Verunreinigungen chromatographiert, indem sie durch eine 5,0 x 85,0 cm - Säule geführt wurde, die mit Sephadex G-200 gepackt und zuvor mit der oben beschriebenen Phosphat-Salz-Pufferlösung ins Gleichgewicht gebracht war, und zwar bei einer Strömungsgeschwindigkeit nach oben von 60 ml/h. Das Eluat aus der Säule wurde in 10 ml-Fraktionen aufgefangen. Die Fraktionen (insgesamt 150) wurden auf ihren Proteingehalt und ihre Radioaktivität

geprüft. Die Fraktionen 100 bis 120, die als den höchsten AFP-Gehalt aufweisend ermittelt wurden, wurden gesammelt und der Rest verworfen.

Ionenaustauschcellulose-Chromatographie

Ein Mischbett-Kationenaustauscher (Carboxymethylcellulose)/Anionenaustauscher (Diäthylaminoäthylcellulose)-Medium wurde mit 0,1 m Ammoniumacetat bei pH 6,25 ins Gleichgewicht gebracht und unter Eigengewicht in eine 2,5 x 13,0 cm-Säule gepackt. Die vereinigten Fraktionen aus der vorhergehenden Stufe wurden mit dem Ammoniumacetatpuffer ins Gleichgewicht gebracht und mit 60 ml/h auf die Säule gebracht. Nach Zugabe der vereinigten Fraktionen wurden 150 ml reiner Puffer auf die Säule gebracht. Dann wurde ein linearer Salzgradient durch allmähliches Erhöhen des Anteils eines Puffers aus 0,5 m Natriumchlorid in 0,1 m Ammoniumacetat vom pH 6,25 in dem Strom reinen Puffers eingestellt. Mit zunehmendem Salzanteil im Pufferstrom werden die Proteine aus der Säule entsprechend ihrem elektrostatischen Bindungsvermögen entfernt. Fraktionen von 3 ml wurden aufgefangen und auf ihren Proteingehalt und ihre Radioaktivität wie bei der vorangegangenen Stufe überprüft. Die AFP-haltigen Fraktionen wurden gesammelt und vereinigt.

Präparative Acrylamid-Elektrophorese

Die vereinigten Fraktionen aus der vorangegangenen Stufe wurden gegen entionisiertes Wasser dialysiert und lyophilisiert. Das erhaltene Pulver wurde in 2,0 ml eines Gemischs aus einem Teil einer 40 gew.-%igen wäßrigen Lösung von Saccharose mit einer Spur an Bromphenolblau und 3 Teilen Elektrophorese-Puffer mit jeweils 6,0 g Tris, d.h., 2-Amino-2-(hydroxymethyl)-1,3-propandiol, pro l und 28,8 g Glycin, pH 8,3,gelöst.

Ein Buchler Polyslab (Buchler Instruments, Inc.) mit einer vertikalen 170 mm x 3,0 mm-Platte aus 7 % auflösendem Polyacrylamidgel (engporig), darauf ein 10 mm x 3,0 mm konzentrierendes Polyacrylamidgel (großporig) wurde mit dem Elektrophoresepuffer ins Gleichgewicht gebracht. Die die Probe enthaltende Lösung wurde auf die Platte gebracht und 24 h unter konstanten Strom von 40 mA gesetzt. Während der Elektrophorese sammelten sich die Proteine in der Probe anfangs als feine Linie in dem konzentrierenden Gel, verteilten sich dann allmählich und schieden sich in dem auflösenden Gel aufgrund der Unterschiede im Molekulargewicht, der Größe und der Ladung ab.

Nach beendeter Elektrophorese wurde das auflösende Gel in 0,5 cm-Streifen quer zur Richtung zwischen Kathode und Anode geschnitten. Die Scheiben wurden dann mit 0,05 m Boratpuffer, pH 8,4, eluiert. Die Eluate wurden durch Doppelimmunodiffusion und RIT-Technik auf Radioaktivität und AFP überprüft. Die hochgereinigtes AFP enthaltenden Eluate wurden durch Ultrafiltration auf etwa 3 ml aufkonzentriert.

## Beispiel 2

Einzelne Serumproben von Affen mit einem chemisch induzierten Hepatom wurden bei -20°C gelagert. Die AFP-Konzentration der Proben wurde zu 0,6 bis 3,2 mg/ml nach der RIT-Arbeitsweise bestimmt. Die Proben wurden aufgetaut und 1 h bei 100.000 UpM zentrifugiert. Mit einem durch RIT als Grundlage erhaltenen Maximalwert wurde die überstehende Flüssigkeit auf einen Gehalt von 0,08 mg AFP/ml mit 0,1 m Natriumphosphatpuffer mit 0,02 Gew.-% Natriumazid, pH 7,0, verdünnt.

10 ml-Portionen des verdünnten Affenserums (mit insgesamt 0,8 mg AFP-RIT-Aktivität) wurden durch ein automatisiertes Rückführ-Chromatographiesystem gemäß der Arbeitsweise des Beispiels 1 geleitet. Die adsorbierten Fraktionen wurden konzentriert und dann an einer Adsorptionssäule mit immobilisiertem Reaktivfarbstoff, d.h. einer Adsorptionssäule mit Sepharose-Blaudextran gemäß der Arbeitsweise des Beispiels 1, chromatographiert.

Die nicht-adsorbierte Fraktion der Arbeitsweise mit immobilisiertem Reaktivfarbstoff wurde dialysiert und lyophilisiert. Das erhaltene Pulver wurde in 2,0 ml Saccharosebromphenolblau-Elektrophoresepuffer, wie in Beispiel 1, gelöst und der präparativen Polyacrylamid-Elektrophorese unterworfen. Die Eluate wurden durch Ultrafiltration auf 3 ml mit hochgereinigtem AFP aufkonzentriert. Es wurde eine spezifische Aktivität von über 0,94 erzielt.

RAN 4093/36

## Patentansprüche

1. Verfahren zur Herstellung gereinigten $\alpha_1$-Fetoproteins, gekennzeichnet durch folgende Schritte:

   a) Puffern einer Probe eines Materials aus einer biologischen Quelle, das dafür bekannt ist, $\alpha_1$-Fetoprotein zu enthalten, auf einen pH von etwa 7,

   b) Affinitätschromatographie der gepufferten Probe durch Hindurchführen durch eine Säule mit einem für $\alpha_1$-Fetoprotein spezifischen Antikörper, der auf einem festen Träger unbeweglich verankert ist, und Eluieren des gebundenen $\alpha_1$-Fetoproteins durch Behandeln der Säule mit einem geeigneten Desorptionsmittel,

   c) Entfernen praktisch des gesamten, im Eluat der Stufe b) vorhandenen Albumins mit Hilfe der Adsorptionschromatographie durch Hindurchleiten durch eine Säule, die einen immobilisierten Reaktivfarbstoff-Adsorber enthält,

d) Dialysieren der das nicht-adsorbierte $\alpha_1$-Fetoprotein enthaltenden Lösung der Stufe c),

e) Lyophilisieren der dialysierten Lösung der Stufe d),

f) Lösen des in Stufe e) gebildeten Pulvers in einem geeigneten Puffer und Polyacrylamid-Gelelektrophorese der erhaltenen Lösung.

2. Verfahren nach Anspruch 1, dadurch gekennzeichnet, daß als Ausgangsmaterial Affen-Hepatomserum verwendet wird.

3. Verfahren nach Anspruch 1, dadurch gekennzeichnet, daß nach den Stufen a) bis d)

e) hochmolekulare Proteinverunreinigungen aus der dialysierten Lösung der Stufe d) durch Gelfiltrationschromatographie unter Verwendung eines hydrophilen, wasserunlöslichen, vernetzten Dextranpolymergels als Filtermaterial entfernt werden,

f) das $\alpha_1$-Fetoprotein enthaltende Eluat aus der Stufe e) zur Entfernung von Proteinverunreinigungen mit Hilfe der Ionenaustauschchromatographie unter Verwendung eines Gemischs kationischen und anionischen Austauscherharzes behandelt wird,

g) das $\alpha_1$-Fetoprotein enthaltende Eluat aus der Stufe f) dialysiert wird,

h) die dialysierte Lösung aus der Stufe g) lyophilisiert wird und

i) das in Stufe h) gebildete Pulver in einem geeigneten Puffer gelöst und die Lösung der Polyacrylamid-Gelelektrophorese unterworfen wird.

0001812

4. Verfahren nach Anspruch 3, dadurch gekennzeichnet, daß als Ausgangsmatrial Nabelschnurblut oder Nabelschnurserum verwendet wird.

5. Radioimmuntest auf $\alpha_1$-Fetoprotein in einer unbekannten Probe, gekennzeichnet durch folgende Schritte:
   a) Bilden eines Komplexes des $\alpha_1$-Fetoproteins in der unbekannten Probe mit einem Antiserum,
   b) Zugabe radioaktiv markierten $\alpha_1$-Fetoproteins,
   c) Entfernen des anfallenden Komplexes aus der Lösung und
   d) Messen der aufgenommenen Menge an radioaktiv markiertem $\alpha_1$-Fetoprotein gegen einen Standard, wobei als radioaktiv markiertes $\alpha_1$-Fetoprotein erfindungsgemäß gereinigtes und mit $^{125}I$ radioaktiv markiertes $\alpha_1$-Fetoprotein verwendet wird.

6. Verwendung des gemäß einem der Ansprüche 1 bis 4 gereinigten $\alpha_1$-Fetoproteins zur Herstellung von Reagentien für einen Radioimmuntest zur Bestimmung von $\alpha_1$-Fetoprotein.

7. Radioaktiv jodiertes $\alpha_1$-Fetoprotein zur Verwendung in einem Human-Radioimmuntest mit nach dem Verfahren gemäß einem der Ansprüche 1 bis 4 gereinigtem und mit $^{125}I$ radioaktiv markiertem $\alpha_1$-Fetoprotein.

8. Reagenssatz zur Bestimmung von $\alpha_1$-Fetoprotein mit nach dem Verfahren gemäß einem der Ansprüche 1 bis 4 gereinigtem und mit $^{125}I$ radioaktiv markiertem $\alpha_1$-Fetoprotein.

0001812

Europäisches Patentamt

**EUROPÄISCHER RECHERCHENBERICHT**

Nummer der Anmeldung

EP 78 101 243.0

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| - | Chemical Abstracts Band 77, Nr. 3, 1972 Columbus, Ohio, USA<br>F. LEHMANN et al "Purification, crystallization, biochemical properties, and clinical significance of α-fetoprotein"<br>Seite 202, Spalte 2, Abstract Nr. 15818m<br>& Verh. Deut. Ges. Inn. Med. Band 77, 1971, Seiten 760-764<br>-- | 1,3 | C 07 G 7/00<br>G 01 N 33/16 |
| | | | RECHERCHIERTE SACHGEBIETE (Int. Cl.²) |
| - | Chemical Abstracts Band 85, Nr. 3 1976 Columbus, Ohio, USA<br>J.L. YOUNG et al "Purification and radioimmunoassay of human alpha -1-fetoprotein: the effect of aggregates on the radioimmunoassay"<br>Seite 304, Spalte 2, Abstract Nr. 16687v<br>& Clin. Chim. Acta Band 69, Heft 1, 1976, Seiten 11-20<br>--<br>./.. | 1,3, 5-8 | C 07 G 7/00<br>G 01 N 33/16 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 04-01-1979 | KNAACK |

Europäisches Patentamt

**EUROPÄISCHER RECHERCHENBERICHT**

Nummer der Anmeldung

EP 78 101 243.0

Seite 2

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| - | Chemical Abstracts Band 87, Nr. 7 1977 Columbus, Ohio, USA C. LAMAR Jr. et al "Purification of α-fetoprotein from rat amniotic fluid by gel filtration" Seite 171, Spalte 2, Abstract Nr. 49594g & J. Chromatogr. Band 137, Heft 2, 1977, Seiten 471-474 -- | 1 | |
| A | US - A 3 901 870 (H. HAUPT et al) * Beispiel * -- | 1 | RECHERCHIERTE SACHGEBIETE (Int. Cl.²) |
| P | Chemical Abstracts Band 88, Nr. 7 1978 Columbus, Ohio, USA P. GOLD et al "Physicochemical approach to the purification of human $\alpha_1$-fetoprotein from the ascites fluid of a hepatomabearing patient" Seite 240, Spalte 1, Abstract Nr. 47086 & Cancer Res. Band 38 Heft 1, 1978, Seiten 6-12 -- | 1, 3 | |

./..

Europäisches Patentamt

# EUROPÄISCHER RECHERCHENBERICHT

Nummer der Anmeldung

EP 78 101 243.0

- Seite 3 -

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| D | Chemical Abstracts Band 85, Nr. 13 1976 Columbus, Ohio, USA<br>S.T. TWOMEY et al "Purification of $\alpha_1$-fetoprotein"<br>Seite 244, Spalte 1,<br>Abstract Nr. 89532a<br>& Clin. Chem. Band 22, Heft 8, 1976, Seiten 1306-1309 | 1, 3 | |
| | | | RECHERCHIERTE SACHGEBIETE (Int. Cl.²) |

EPA Form 1503.2 06.78